(19) Europäisches Patentamt
European Patent Office
Office européen des brevets

(11) Veröffentlichungsnummer: **0 362 555**
**A1**

(12) **EUROPÄISCHE PATENTANMELDUNG**

(21) Anmeldenummer: **89116093.9**

(22) Anmeldetag: **31.08.89**

(51) Int. Cl.[5]: **C07D 241/08 , C07D 487/04 , C07D 487/10 , A61K 31/495 , //(C07D487/04,241:00,209:00), (C07D487/10,241:00,209:00)**

(30) Priorität: **03.09.88 DE 3830096**

(43) Veröffentlichungstag der Anmeldung:
**11.04.90 Patentblatt 90/15**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Nickel, Wolf-Ulrich, Dr.**
**Robert-Stolz-Strasse 120**
**D-6232 Bad Soden am Taunus(DE)**
Erfinder: **Henning, Rainer, Dr.**
**Rotenhofstrasse 31**
**D-6234 Hattersheim am Main(DE)**
Erfinder: **Rüger, Wolfgang, Dr.**
**Königsteinerstrasse 13**
**D-6238 Hofheim am Taunus(DE)**
Erfinder: **Lerch, Ulrich, Dr.**
**Voderwart 24**
**D-6238 Hofheim am Taunus(DE)**
Erfinder: **Urbach, Hansjörg, Dr.**
**Le Lavandoustrasse 41**
**D-6242 Kronberg/Taunus(DE)**
Erfinder: **Hock, Franz, Dr.**
**Altstadt 19**
**D-6110 Dieburg(DE)**
Erfinder: **Wiemer, Gabriele, Dr.**
**Ernst-Moritz-Arndt Strasse 21**
**D-6242 Kronberg/Taunus(DE)**

(54) **Piperazindione mit psychotroper Wirkung.**

(57) Die Erfindung betrifft Verbindungen der Formel I

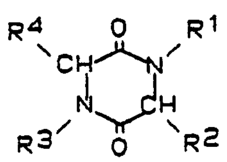

(I),

worin $R^1$, $R^2$ und $R_4$ für gegebenenfalls substituierte aliphatische, alicyclische, alicyclisch-aliphatische, aromatische, araliphatische, heteroaromatische oder heteroaromatisch-aliphatische Reste stehen, wobei $R^2$, falls nicht schon von den vorstehenden Definitionen umfaßt, die erforderlichenfalls geschützte Seitenkette einer natürlich vorkommenden $\alpha$-Aminosäure bedeutet, $R^4$ zusätzlich für

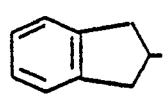

stehen kann, $R^3$ einen gegebenenfalls substituierten aliphatischen, alicyclischen, aromatischen oder araliphatischen Rest bedeutet, oder $R^3$ und $R^4$ zusammen mit den sie tragenden Atomen ein mono-, bi- oder

EP 0 362 555 A1

tricyclisches heterocyclisches Ringsystem bilden,
diese enthaltende Mittel und deren Verwendung als Arzneimittel mit psychotroper Wirkung zur Prophylaxe von Störungen im zentralen Nervensystem, insbesondere mit nootroper Wirkung zur Behandlung cognitiver Dysfunktionen.

## Piperazindione mit psychotroper Wirkung

Die Erfindung betrifft neue 1,4-Piperazin-2,5-dione, diese enthaltende Mittel und deren Verwendung als Arzneimittel mit psychotroper Wirkung zur Behandlung und Prophylaxe von Störungen im zentralen Nervensystem, insbesondere mit nootroper Wirkung zur Behandlung cognitiver Dysfunktionen.

Es ist bekannt, daß (3S)-3-(2-Methylpropyl)-1,4-piperazin-2,5-dion (cyclo(Leu-Gly)) das zentrale Nervensystem beeinflußt, vor allem in der Toleranzverminderung zu Morphin (J. Pharmakol. Exp. Ther. 218 (1981) 404, WP 8000-216) und als Antagonist in der Puromycin-induzierten Amnesie (Pharmakol., Biochem. Behav. 10 (1979) 787; Pharmakol., Biochem. Behav. 8 (1978) 93; Jap. Pat. J5 4073-133).
Weiterhin sind Piperazin-2,5-dione z.B. als Analgetika (J5 8083-682-A, J5 9073-574-A), als Antiulcera (EP 008 186, US 3,976,773) und als PAF-Antagonisten (EP 181 152) beschrieben.

Der Erfindung liegt die Aufgabe zugrunde, neue Verbindungen mit antiamnestischer Wirkung zu finden.

Diese Aufgabe wird erfindungsgemäß gelöst durch die Verbindungen der Formel I

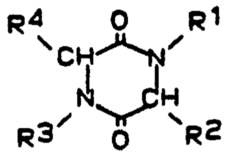

(I),

in welcher
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^3$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen bedeutet,
$R^4$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatischen-($C_1$-$C_8$)-aliphatischen Rest mit 5 - 12 Ringatomen bedeutet, oder
für

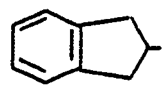

steht oder

$R^3$ und $R^4$ zusammen mit den sie tragenden Atomen ein mono,-bi- oder tricyclisches heterocyclisches Ringsystem mit 3 bis 15 Ring-C-Atomen bilden.

Unter einem gegebenenfalls substituierten aliphatischen Rest versteht man einen aliphatischen acyclischen Rest, d.h. einen Rest mit einer offenen, geraden oder verzweigten Kohlenstoffkette, wie beispielsweise Alkyl, Alkenyl, Alkinyl und entsprechende mehrfach ungesättigte Reste. Er ist vorzugsweise unsubstituiert oder beispielsweise durch Hydroxy, Alkoxy, Halogen, Amino, Alkanoylamino, Alkoxycarbonylamino, Arylalkoxycarbonylamino, Arylalkylamino, Alkylamino, Dialkylamino, Alkylthio, Arylthio, Carboxy, Carbamoyl, Alkoxycarbonyl, Alkanoyloxy, Alkoxycarbonyloxy, Aroyloxy oder Aryloxycarbonyloxy monosubstituiert.

Ein gegebenenfalls substituierter alicyclischer Rest und der über eine offene Kohlenstoffkette verknüpfte entsprechende gegebenenfalls substituierte alicyclisch-aliphatische Rest ist ein vorzugsweise mono- bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Er ist vorzugsweise unsubstituiert. Mehrere Ringe als Komponenten eines solchen Restes sind kondensiert, spiroverknüpft oder isoliert. Beispiele für solche Reste sind Cycloalkyl, Cycloalkenyl, Cycloalkylalkyl, Bicycloalkyl, Tricycloalkyl und von mono-, bi- oder oligocyclischen Terpenen abgeleitete Reste, wie Menthyl, Isomenthyl, Bornanyl, Bornyl, Caranyl, Epibornyl, Epiisobornyl, Isobornyl, Menthanyl, Neomenthyl, Neoisomenthyl, Pinanyl, Thujanyl; sie sind vorzugsweise unsubstituiert (aliphatische Seitenketten sind nach vorliegender Definition keine Substituenten).

Ein gegebenenfalls substituierter aromatischer Rest ist vorzugsweise Aryl wie Phenyl, Biphenylyl oder Naphthyl, das gegebenenfalls wie auf Seite 8 bei Aryl angegeben mono-, di- oder trisubstituiert ist. Von Aryl abgeleitete Reste, wie Aralkyl, Aryloxy, Arylthio oder Aroyl, vorzugsweise Benzoyl, können wie Aryl substituiert sein.

Ein gegebenenfalls substituierter heteroaromatischer Rest ist vorzugsweise ein aromatischer mono- bzw. bicyclischer Heterocyclen-Rest mit 5 bis 7 bzw. 8 bis 12, bevorzugt bis 10 Ringatomen, wovon 1 bis 2 Ringatome Schwefel- oder Sauerstoffatome und/oder wovon 1 bis 4 Ringatome Stickstoffatome darstellen, wie beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein. Ein heteroaromatischer Rest und der entsprechende heteroaromatisch-aliphatische Rest können wie unten definiert, substituiert sein.

Unter einem gegebenenfalls substituierten araliphatischen Rest werden insbesondere Aralkylreste wie Arylalkyl, Diarylalkyl, Indanyl oder Fluorenyl verstanden, worin Aryl wie unten definiert ist und in der dort angegebenen Weise substituiert sein kann.

$R^3$ und $R^4$ kann mit den diese tragenden Atomen ein mono-, bi- oder tricyclisches heterocyclisches Ringsystem mit 3 bis 15 Ring-C-Atomen bilden, das im Ring vorzugsweise bis zu 2 S-Atome und bis zu 2 N-Atome, insbesondere bis zu 1 S-Atom aufweist.

Als solche Ringsysteme kommen insbesondere jene aus der folgenden Gruppe in Betracht:
Pyrrolidin (O); Thiazolidin (R); Tetrahydroisochinolin (A); Decahydroisochinolin (B); Octahydroindol (C); Indolin (Q); Octahydrocyclopenta[b]pyrrol (D); 2-Azaspiro[4.5]decan (E); 2-Azaspiro[4.4]-nonan (F); Spiro[(bicyclo[2.2.1]heptan)-2,3′-pyrrolidin] (G; Spiro[(bicyclo[2.2.2]octan)-2,3′- pyrrolidin] (H); 2-Azatricyclo[4.3.0.1$^{6,9}$]decan (I); Decahydrocyclohepta[b]pyrrol (J); Octahydroisoindol (K); Octahydrocyclopenta[c]pyrrol (L); 2,3,3a,4,5,7a-Hexahydroindol (M); 2-Axa-bicyclo[3.1.0]hexan (N); 1,2,3,3a,4,6a-Hexahydrocyclopenta[b]pyrrol (P), die alle gegebenenfalls substituiert sein können. Pyrrolidin (O) und Thiazolidin (R) können z.B. durch ($C_6$-$C_{12}$)-Aryl (Phenyl, 2-Hydroxyphenyl, u.a.), ($C_6$-$C_{12}$)-Arylmercapto (wie Phenylmercapto) oder ($C_3$-$C_7$)-Cycloalkyl (wie Cyclohexyl) monosubstituiert sein. Entsprechendes gilt für die anderen Ringsysteme. Bevorzugt sind jedoch die unsubstituierten Systeme.

Bei Verbindungen der Formel I, die mehrere chirale Atome besitzen, kommen alle möglichen Diastereomere als Racemate oder Enantiomere, oder Gemische verschiedener Diastereomere in Betracht.

Die in Betracht kommenden heterocyclischen Ringsysteme weisen die folgenden Strukturformeln auf.

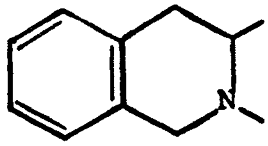

A

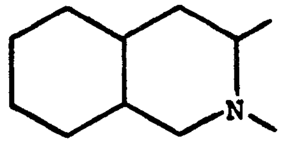

B

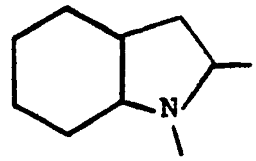

C

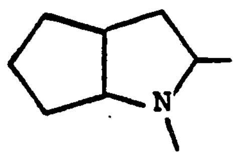

D

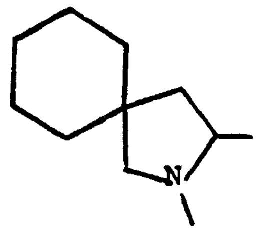

E

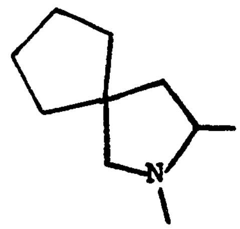

F

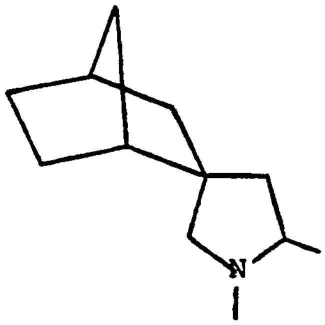

G

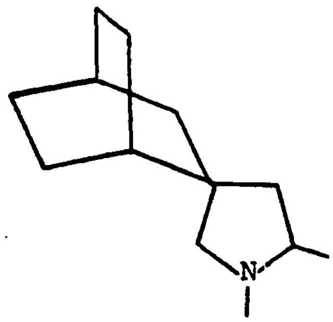

H

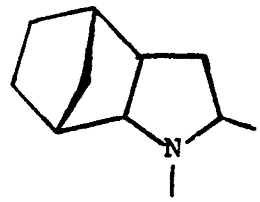

I

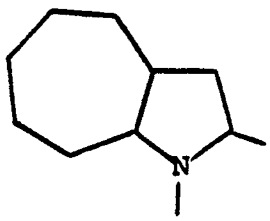

J

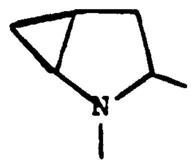

K

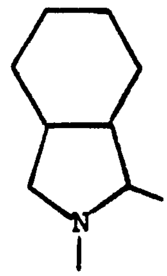

L

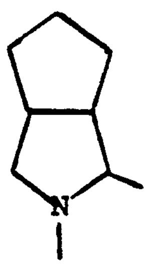

M

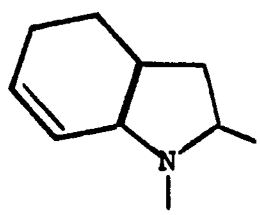

N

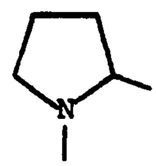

O

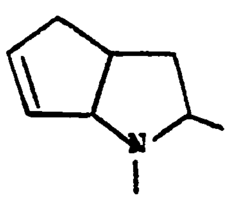

P

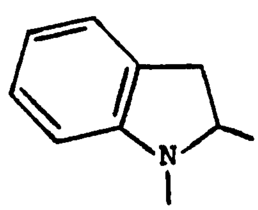

Q

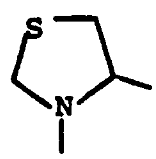

R

Eine bevorzugte Ausführungsform ist gekennzeichnet durch Verbindungen der Formel I, in welcher

$R^1$ Wasserstoff;

$(C_1-C_{18})$-Alkyl;

einen aliphatisch acyclischen Rest der Formel $C_aH_{(2a+b-1)}$, worin Doppelbindungen, falls ihre Zahl 1 übersteigt, nicht kumuliert sind, a für eine ganze Zahl 2 bis 18 und b für eine ganze Zahl 2 bis a stehen;

einen mono-, di- oder tricyclischen, nicht aromatischen, gegebenenfalls verzweigten Kohlenstoffrest der Formel $C_cH_{(2c-d-1)}$ bedeutet, worin c für eine ganze Zahl 3 bis 20 und d für eine ganze Zahl 0 bis (c-2) stehen;

$(C_6-C_{12})$-Aryl, das durch $(C_1-C_8)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl mono-, di- oder trisubstituiert sein kann;

Amino-$(C_1-C_8)$-alkyl;

$(C_1-C_4)$-Alkanoylamino-$(C_1-C_8)$-alkyl;

$(C_7-C_{13})$-Aroylamino-$(C_1-C_8)$-alkyl;

$(C_1-C_4)$-Alkoxycarbonylamino-$(C_1-C_8)$-alkyl;

$(C_6-C_{12})$-Aryl-$(C_1-C_4)$-alkoxycarbonylamino-$(C_1-C_8)$-alkyl;

$(C_6-C_{12})$-Aryl-$(C_1-C_4)$-alkylamino-$(C_1-C_8)$-alkyl;

$(C_1-C_4)$-Alkylamino-$(C_1-C_8)$-alkyl;

Di-$(C_1-C_4)$-alkylamino-$(C_1-C_8)$-alkyl;

Guanidino-$(C_1-C_8)$-alkyl;

$(C_1-C_4)$-Alkylthio-$(C_1-C_8)$-alkyl;

$(C_6-C_{12})$-Arylthio-$(C_1-C_8)$-alkyl oder $(C_6-C_{12})$-Aryloxy-$(C_1-C_8)$-alkyl, die beide im Arylteil wie oben beschrieben substituiert sein können;

Carboxy-$(C_1-C_{17})$-alkyl, $(C_1-C_4)$-Alkoxycarbonyl-$(C_1-C_{17})$-alkyl, Carbamoyl-$(C_1-C_{17})$-alkyl, $(C_1-C_4)$-Mono-oder-di-alkylcarbamoyl-$(C_1-C_{17})$-alkyl, wobei Alkyl gegebenenfalls durch $(C_6-C_{12})$-Aryl substituiert ist, bedeutet;

$R^2$ Wasserstoff,

$(C_1-C_{18})$-Alkyl;

einen aliphatischen acyclischen Rest der Formel $C_aH_{(2a-b+1)}$, worin Doppelbindungen, falls ihre Zahl 1 übersteigt, nicht kumuliert sind, a für eine ganze Zahl 2 bis 18 und b für eine ganze Zahl 2 bis a stehen;

einen mono-, di-, tri-, tetra- oder pentacyclischen, nicht aromatischen, gegebenenfalls verzweigten Kohlenwasserstoffrest der Formel $C_cH_{(2c-d-1)}$, worin c für eine ganze Zahl 3 bis 20 und d für eine gerade Zahl 0 bis (c-2) stehen;

$(C_6-C_{12})$-Aryl;

$(C_6-C_{12})$-Aryl-$(C_1-C_8)$-alkyl oder $(C_7-C_{13})$-Aroyl-$(C_1-C_8)$-alkyl, die im Arylteil wie oben beschrieben substituiert sein können;

mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroarylteil wie oben bei Aryl beschrieben substituiert sein können; oder, falls noch nicht umfaßt, die gegebenenfalls geschützte Seitenkette einer natürlich vorkommenden α-Aminosäure der Formel $R^1$-CH($NH_2$)-COOH bedeutet; und

$R^3$ und $R^4$ zusammen mit den sie tragenden Atomen ein mono-, bi- oder tricyclisches heterocyclisches Ringsystem mit 3 bis 15 Ring-C-Atomen bilden.

Besonders bevorzugt sind Verbindungen der Formel I, in welcher

$R^1$ Wasserstoff;

$(C_1-C_8)$-Alkyl;

$(C_2-C_6)$-Alkenyl;

$(C_3-C_9)$-Cycloalkyl;

$(C_6-C_{12})$-Aryl, das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann;

mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen, das wie oben bei Aryl beschrieben substituiert sein kann;

Amino-$(C_1-C_4)$-alkyl;

$(C_1-C_4)$-Alkanoylamino-$(C_1-C_4)$-alkyl;

$(C_7-C_{13})$-Aroylamino-$(C_1-C_4)$-alkyl;

($C_1$-$C_4$)-Alkoxycarbonylamino-($C_1$-$C_4$)-alkyl;
($C_6$-$C_{12}$)-Aryl-($C_1$-$C_4$)-alkoxycarbonylamino-($C_1$-$C_4$)-alkyl;
($C_6$-$C_{12}$)-Aryl-($C_1$-$C_4$)-alkylamino-($C_1$-$C_4$)-alkyl;
($C_1$-$C_4$)-Alkylamino-($C_1$-$C_4$)-alkyl;
Di-($C_1$-$C_4$)-alkylamino-($C_1$-$C_4$)-alkyl;
Guanidino-($C_1$-$C_4$)-alkyl;
($C_1$-$C_4$)-Alkylthio-($C_1$-$C_4$)-alkyl;
($C_6$-$C_{12}$)-Arylthio-($C_1$-$C_4$-alkyl, das im Arylteil wie oben bei Aryl beschrieben substituiert sein kann;
Carboxy-($C_1$-$C_4$)-alkyl;
Carbamoyl-($C_1$-$C_4$)-alkyl;
($C_1$-$C_4$)-Alkoxy-carbonyl-($C_1$-$C_4$)-alkyl;
($C_6$-$C_{12}$)-Aryloxy-($C_1$-$C_4$)-alkyl, das im Arylteil wie oben bei Aryl beschrieben substituiert sein kann, bedeutet,
$R^2$ Wasserstoff;
($C_1$-$C_6$)-Alkyl;
($C_2$-$C_6$)-Alkenyl;
($C_2$-$C_6$)-Alkinyl;
($C_3$-$C_9$)-Cycloalkyl;
($C_5$-$C_9$)-Cycloalkenyl;
($C_3$-$C_9$)-Cycloalkyl-($C_1$-$C_4$)-alkyl;
($C_5$-$C_9$)-Cycloalkenyl-($C_1$-$C_4$)-alkyl;
gegebenenfalls teilhydriertes ($C_6$-$C_{12}$)-Aryl, das wie oben bei $R^1$ beschrieben substituiert sein kann;
($C_6$-$C_{12}$)-Aryl-($C_1$-$C_4$)-alkyl oder ($C_7$-$C_{13}$)-Aroyl-($C_1$ oder $C_2$)-alkyl, die beide wie das vorstehende Aryl substituiert sein können;
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder die gegebenenfalls geschützte Seitenkette einer natürlich vorkommenden α-Aminosäure $R^1$-CH($NH_2$)-COOH bedeutet; und
$R^3$ und $R^4$ die oben angegebene Bedeutung haben.

Insbesondere seien Verbindungen der Formel I genannt, in welcher
$R^1$ Wasserstoff; ($C_1$-$C_8$)-Alkyl; ($C_2$-$C_6$)-Alkenyl; ($C_3$-$C_9$)-Cycloalkyl; Amino-($C_1$-$C_4$)-alkyl; ($C_2$-$C_5$)-Acylamino-($C_1$-$C_4$)-alkyl; ($C_7$-$C_{13}$)-Aroylamino-($C_1$-$C_4$)-alkyl; ($C_1$-$C_4$)-Alkoxycarbonylamino-($C_1$-$C_4$)-alkyl; ($C_1$-$C_4$)-Alkoxycarbonyl-($C_1$-$C_{10}$)-alkyl, Carbamoyl-($C_1$-$C_{10}$)-alkyl, ($C_1$-$C_4$)-Mono- oder-di-alkylcarbamoyl-($C_1$-$C_{10}$)-alkyl, wobei Alkyl gegebenenfalls durch Phenyl substituiert ist;
($C_6$-$C_{12}$)-Aryl-($C_1$-$C_6$)-alkyl; ($C_6$-$C_{12}$)-Aryl-($C_1$-$C_4$)-alkoxycarbonylamino-($C_1$-$C_4$)-alkyl; ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann;
insbesondere Wasserstoff, Methyl, Ethyl, tert. Butoxycarbonylamino-($C_1$-$C_4$)-alkyl, Benzoyloxycarbonylamino-($C_1$-$C_4$)-alkyl, Carboxy-($C_1$-$C_{10}$)-alkyl, ($C_1$-$C_4$)-Alkoxycarbonyl-($C_1$-$C_{10}$)-alkyl bedeutet;
$R^2$ Wasserstoff; ($C_1$-$C_6$)-Alkyl, das gegebenenfalls durch Amino, ($C_1$-$C_6$)-Acylamino oder Benzoylamino substituiert sein kann; ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann; ($C_6$-$C_{12}$)-Aryl-($C_1$ - $C_4$)-alkyl oder ($C_7$-$C_{13}$)-Aroyl-($C_1$-$C_2$)-alkyl, die beide wie vorstehend definiert im Arylrest substituiert sein können; ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen; oder eine Seitenkette einer natürlich vorkommenden, gegebenenfalls geschützten α-Aminosäure; insbesondere aber Wasserstoff; ($C_1$-$C_3$)-Alkyl; ($C_2$ oder $C_3$)-Alkenyl; die gegebenenfalls geschützte Seitenkette von Lysin; Benzyl, 4-Methoxybenzyl, 4-Ethoxybenzyl, Phenethyl, 4-Amino-butyl oder Benzoylmethyl bedeutet, und
$R^3$ und $R^4$ zusammen mit den sie tragenden Atomen ein bi-oder tricyclisches heterocyclisches Ringsystem aus der Reihe Octohydroindol, Octohydrocyclopenta[b]pyrrol, Spiro[(bicyclo[2.2.2]octan)-2,3′-pyrrolidin], 1,2,3,3a,4,6a-Hexahydrocyclopenta[b]pyrrol,Indolin bilden.

Falls $R^2$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen in der Peptidchemie übliche Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen

bevorzugt ($C_1$-$C_6$)-Alkyl, insbesondere Methyl oder Ethyl in Frage.

Die Erfindung betrifft weiterhin ein Verfahren zur Herstellung einer Verbindung der Formel I, das dadurch gekennzeichnet ist, daß man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, die so erhaltenen Verbindungen intramolekular cyclisiert, Verbindungen der Formel I mit freier(en) Carboxylgruppe(n) gegebenenfalls verestert und die erhaltenen Verbindungen gegebenenfalls in ihre physiologisch verträglichen Salze überführt.

In der genannten Weise kann man beispielsweise Verbindungen der Formel II und III unter Bildung von Verbindungen der Formel IV

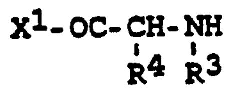

(II)

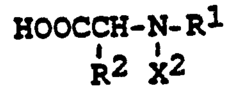

(III)

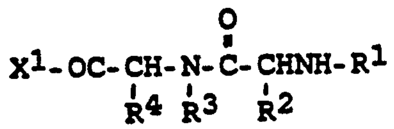

(IV)

umsetzen, worin $R^1$ bis $R^4$ in oben angegebener Weise definiert ist; $X^1$ für ($C_1$-$C_8$)-Alkoxy, ($C_7$-$C_{13}$)-Arylalkoxy, ($C_1$-$C_4$)-Alkylamino, ($C_7$-$C_{13}$)-Arylalkylamino, bevorzugt jedoch für Methoxy, Ethoxy, Benzyloxy, tert.-Butoxy, als temporär eingeführte Schutzgruppe für die nicht an der Reaktion beteiligte Carboxylgruppe, die nach beendeter Reaktion in an sich bekannter Weise abgespalten werden kann; und

$X^2$ für die in der Peptidsynthese bekannten Schutzgruppen für Aminogruppen, (vgl. Houben-Weyl, Meth. d. organ. Chemie Band XV 1 u. 2; Greene, "Protective Groups in Organic Synthesis", New York 1981), wie z.B. tert.-Butoxycarbonyl, Fluorenylmethoxycarbonyl, Benzyloxycarbonyl, die in an sich bekannter Weise nach erfolgter Reaktion wieder abgespalten werden können.

Die Verbindungen der Formel IV können entweder mit der Gruppe $X^1$ oder nach separater Abspaltung von $X^1$ zur Carboxylgruppe in genannter Weise zu den erfindungsgemäßen Verbindungen der Formel I intramolekular cyclisiert werden.

Die Umsetzung dieser Verbindungen kann beispielsweise in Analogie zu bekannten Peptidkupplungsverfahren in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphorsäureanhydriden, Dialkylphosphinsäureanhydriden oder N,N-Succinimidylcarbonate in einem Lösungsmittel wie $CH_3CN$ durchgeführt werden. Aminogruppen in Verbindungen der Formel II können mit Tetraethyldiphosphit aktiviert werden. Die Verbindungen der Formel III können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76 - 136). Die Reaktion wird vorzugsweise zwischen -20 °C und dem Siedepunkt des Reaktionsgemisches durchgeführt.

Die Umsetzung zu Verbindungen der Formel IV kann aber auch thermisch in einem geeigneten organischen Lösungsmittel im Temperaturbereich zwischen 0 °C und dem Siedepunkt des Lösungsmittels erfolgen.

Ebenso können Verbindungen der Formel V und VI in der genannten Weise zu Verbindungen der Formel VII miteinander verknüpft werden und anschließend zu Verbindungen der Formel I cyclisiert werden.

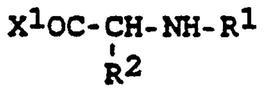

(V)

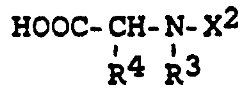

(VI)

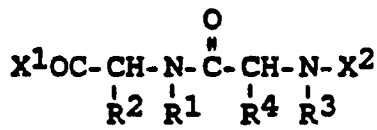

(VII)

Ebenso lassen sich auch Verbindungen der Formel VIII mit Verbindungen der Formel IX unter Bildung von Verbindungen der Formel IV,

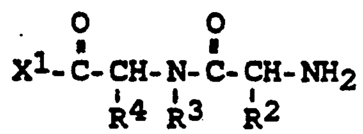

(VIII)

$Y^1-R^1$

(IX)

worin $R^1$, $R^2$, $R^3$, $R^4$ und $X^1$ wie oben definiert sind und $Y^1$ für eine Abgangsgruppe steht. Geeignete Abgangsgruppen sind z.B. Cl, Br, I, Alkylsulfonyloxy oder Arylsulfonyloxy.

Alkylierung dieser Art führt man zweckmäßigerweise in Wasser oder einem organischen Lösungsmittel wie Dichlormethan oder einem weiteren aliphatischen Alkohol (wie Ethanol), Benzylalkohol, Acetonitril, Nitromethan oder Glycolether bei einer Temperatur zwischen -20 °C und dem Siedepunkt des Reaktionsgemisches in Gegenwart einer Base - wie einem Alkalimetallhydroxid oder einem organischen Amin - durch.

Die Herstellung der neuen Verbindungen der allgemeinen Formel I kann beispielsweise auch unter Anwendung dem Fachmann vertrauter Alkylierungsmethoden erfolgen (s. z.B. Houben-Weyl, Methoden der organischen Chemie Band E 5), beispielsweise die Umsetzung einer Verbindung der Formel I, worin mindestens einer der Reste $R^1$ und $R^3$ Wasserstoff bedeutet, mit einem entsprechenden Alkylierungsmittel der allgemeinen Formel IX, wie z.B. einem Alkylhalogenid, unter basischer Katalyse in einem polar protischen oder dipolar aprotischen Lösungsmittel.

Die nootrope Wirkung der erfindungsgemäßen Verbindungen wurde an Mäusen, die ein Körpergewicht von 20 - 25 g besaßen im inhibitory (passive) avoidance test (step-through-Modell) geprüft. Eine modifizierte Form der von J. KOPP, Z. BODANECKY und M.E. JARVIK beschriebenen Testmethode wurde von J. BURES, O. BURESOVA und J. HUSTON in "Techniques and Basic Experiments for the Study of Brain and Behavior", Elsevier Scientific Publishers, Amsterdam (1983) beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie oder die mittels Scopolamin induzierte Amnesie aufzuheben vermag.

Die Versuche wurden nach modifizierten Testmethoden durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß die Scopolamin-induzierte Amnesie im inhibitory avoidance-Test sich mit einer MED (minimal effective Dosis) von 1,0 - 30 mg/kg p.o. aufheben läßt. Die Vergleichssubstanz hat eine MED von ca. 500 - 1000 mg/kg p.o.

Die erfindungsgemäßen Verbindungen haben im allgemeinen nur eine geringe Toxizität und sind aufgrund ihrer pharmakologischen Eigenschaften für die Behandlung cognitiver Dysfunktionen unterschiedlicher Genese, wie sie z.B. bei der Alzheimer'schen Krankheit oder der senilen Demenz auftreten, geeignet.

Die Erfindung umfaßt weiterhin die genannten Verbindungen enthaltende Arzneimittel, Verfahren zu deren Herstellung sowie die Verwendung der erfindungsgemäßen Verbindungen bei der Herstellung von Arzneimitteln, die zur Behandlung und Prophylaxe der vorstehend genannten Krankheiten eingesetzt

werden.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral, rektal oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magensiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

**Beispiel 1**

(3S,5 S,8aS,9aS)-3-Methyl-2H-decahydrocyclopenta[4,5]pyrrolo-[1,2-a]pyrazin-1,4-dion

a) 41,2 g (1S,3S,5S)-2-Azabicyclo[3.3.0]octancarbonsäurebenzylester und 34,8 g N-tert.-Butoxycarbonyl-L-alanin wurden in 680 ml Dimethylformamid mit 116 ml Triethylamin und 170 ml einer 50 %igen Lösung von n-Propylphosphonsäureanhydrid (n-PropPA) in $CH_2Cl_2$ bei -5 °C versetzt und über Nacht bei Raumtemperatur gerührt. Die Reaktionslösung wurde mit 1 l Essigester verdünnt und zweimal mit gesättigter $NaHCO_3$-Lösung, zweimal mit 10 %iger Citronensäurelösung und einmal mit gesättigter NaCl-Lösung gewaschen, mit $MgSO_4$ getrocknet und bis zur öligen Konsistenz im Vakuum eingeengt. Die Ausbeute betrug 65,9 g eines farblosen Öls.

$[\alpha]_D^{20}$ = -52,2° (c = 1 in Methanol)

b) 14,6 g des unter a) beschriebenen 2-(N-tert. Butoxycarbonyl-S-alanyl)-(1S,3S,5S)-2-azabicyclo-[3.3.0]octancarbonsäurebenzylesters wurden bei 0 °C mit 25 ml Trifluoressigsäure dreißig Minuten, anschließend zwei Stunden bei Raumtemperatur gerührt. Die Lösung wurde im Vakuum eingeengt und dreimal nach Zugabe von jeweils 10 ml Toluol erneut eingeengt. Der so erhaltene Rückstand wurde 9 Stunden in 100 ml Essigester unter Rückfluß erhitzt. Nach Abkühlen der Reaktionslösung wurden 4,06 g farbloser Kristalle vom Schmelzpunkt 222 °C erhalten.

$[\alpha]_D^{20}$ = +3,12° (c = 1 in Methanol)

**Beispiel 2**

(3S,5 R,8aR,9aR)-3-Methyl-2H-decahydrocyclopenta[4,5]pyrrolo[1,2-a]pyrazin-1,4-dion

a) 2-(N-tert.Butoxycarbonyl-S-alanyl)-(1R,3R,5R)-2-azabicyclo[3.3.0]octancarbonsäurebenzylester 4,91 g (1R,3R,5R)-2-Azabicyclo[3.3.0]octancarbonsäurebenzylester und 4,12 g N-tert.Butoxycarbonyl-L-alanin wurden in 80 ml absolutem Dimethylformamid gelöst, mit 12,7 ml N-Ethylmorpholin und 20, 2 ml n-

Propylphosphonsäureanhydrid (50 % in $CH_2Cl_2$) bei 0 °C versetzt. Die Reaktionsmischung wurde 5 Stunden bei Raumtemperatur gerührt. Nach Zugabe von 120 ml Essigester wurde die Mischung zweimal mit gesättigter $NaHCO_3$-Lösung, zweimal mit 10 %iger Citronensäurelösung und einmal mit gesättigter Salzlösung gewaschen. Nach Trocknen und Einengen der organischen Phasen verblieben 8,04 g eines viskosen Öls.

$[\alpha]_D^{20}$ = 97,2° (c = 1 in Methanol)

b) 7 g des unter a) erhaltenen Öls wurden in 290 ml Ethanol mit Pd auf Kohle bei Raumtemperatur hydriert. Nach Filtration vom Katalysator und Einengen der Lösung verblieben 5,5 g eines farblosen Feststoffes vom Schmelzpunkt 140 °C.

c) 1 g der unter b) erhaltenen Carbonsäure wurde für 45 Minuten bei 0 °C mit 5 ml Trifluoressigsäure behandelt. Der nach dem Einengen erhaltene feste Rückstand wurde 9 Stunden in iso-Propanol unter Rückfluß erhitzt. Das Lösungsmittel wurde im Vakuum abgezogen und der Rückstand aus Essigester umkristallisiert. Es wurden 435 mg farbloser Kristalle erhalten. Schmelzpunkt 214 °C.

**Beispiel 3**

(3S,5 S,8aS,9aS)-3-Cyclohexylmethyl-2H-decahydrocyclopenta[4,5]pyrrolo-[1,2-a]pyrazin-1,4-dion

a) 5,7 g N-tert.Butoxycarbonyl-L-Cyclohexylalanin und 5,5 g (1S,3S,5S)-2-Azabicyclo[3.3.0]-octancarbonsäurebenzylester wurden in 55 ml $CH_2Cl_2$/DMF 10:1 gelöst und bei 0 °C mit 15 ml N-Ethylmorpholin und 24 ml n-Propylphosphonsäureanhydrid (50 %ig in $CH_2Cl_2$) versetzt. Die Reaktionslösung wurde über Nacht bei Raumtemperatur gerührt, mit 150 ml $CH_2Cl_2$ verdünnt und wurde mit gesättigter $NaHCO_3$, 10 %iger Citronensäure und gesättigter Salzlösung extrahiert. Nach Trocknen und Einengen der organischen Phase wurde ein braunes Öl erhalten, das säulenchromatographisch (Kieselgel, Cyclohexan/Essigester) gereinigt wurde. Die Ausbeute betrug 7,9 g farbloses Öl.

$[\alpha]_D^{20}$ = -29,9° (c = 1 in Methanol)

b) 1,5 g des unter a) erhaltenen Öls wurden in 7 ml mit Chlorwasserstoff gesättigtem Dimethoxyethan gelöst und 0,5 Stunden bei Raumtemperatur gerührt. Die Lösung wurde bis zur Trockne eingeengt. Der Rückstand wurde in 20 ml iso-Propanol aufgenommen und 6 Stunden unter Rückfluß erhitzt. Nach dem Abkühlen wurden aus der Lösung 450 mg farblose Kristalle durch Absaugen gewonnen. Schmelzpunkt 239 °C.

**Beispiel 4**

(3S,5 S,8aS,9aR)-3-Methyl-2N-(3-phenylpropyl)-2H-decahydrocyclopenta[4,5]pyrrolo[1,2-a]pyrazin-1,4-dion

1 g des in Beispiel 1 beschriebenen Piperazin-2,5-dions wurden bei 0 °C in 20 ml Dimethylformamid gelöst und mit 0,56 g Kalium-tert.butylat versetzt. Nach 45 Minuten Reaktionszeit war eine klare gelbe Lösung entstanden, zu der 0,73 ml 3-Phenylpropylbromid getropft wurden. Nach 2 Stunden bei 0 °C und Aufwärmen auf Raumtemperatur wurde die Lösung auf Wasser gegossen und dreimal mit Essigester extrahiert, die organische Phase getrocknet und eingeengt. Nach chromatographischer Reinigung (Kieselgel, Cyclohexan/Essigester) wurden 1.04 g farblose Kristalle erhalten. Schmelzpunkt 92 °C.

$[\alpha]_D^{20}$ = +127,5° (c = 1 in Methanol)

**Beispiel 5**

(3S,5S,8aS,9aR)-3-Methyl-2N-octyl-2H-decahydrocyclopenta-[4,5]pyrrolo[1,2-a]pyrazin-1,4-dion

2 g des unter Beispiel 1 beschriebenen Piperazin-2,5-dions wurden in 50 ml DMF gelöst und unter Eiskühlung mit 1,12 g Kalium-tert.butylat versetzt. Nach 40 Minuten wurden 1,66 ml n-Octylbromid zugetropft und die Reaktionsmischung über Nacht gerührt. Die Lösung wurde mit Wasser verdünnt und mehrmalig mit Essigester extrahiert. Nach Trocknen und Einengen der organischen Phase verblieben 3,3 g öliges Rohprodukt. Die chromatographische Reinigung über Kieselgel (Cyclohexan/Essigester) ergab 1,1 g erwünschtes Produkt vom Schmelzpunkt 44-47 °C.

$[\alpha]_D^{20}$ = +156,1° (c = 1 in Methanol)

**Beispiel 6**

(3S,5 S,8aS,9aS)-3-Methyl-2N-(1-S-ethoxycarbonylheptyl)-2H-decahydrocyclopenta[4,5]pyrrolo[1,2-a]-pyrazin-1,4-dion

a) N-(1,R,S-Ethoxycarbonyl-heptyl)-L-alaninbenzylester

7,56 g L-Alaninbenzylester x p-Toluolsulfonsäure werden in 200 ml Dichlormethan gelöst und bei 0 °C mit 8,6 ml Triethylamin und anschließend mit 6,2 g 2-Trifluormethansulfonyloxyoctansäureethylester versetzt und 24 Stunden gerührt. Die Reaktionslösung wurde eingeengt, der Rückstand in Essigester aufgenommen und mit $NH_4Cl$-Lösung und NaCl-Lösung gewaschen, getrocknet mit $MgSO_4$ und eingeengt. Das erhaltene Rohprodukt von 6,2 g wurde säulenchromatographisch (Kieselgel, Cyclohexan/Essigester) in die beiden diastereomeren Produkte getrennt.
Das unpolarere Diastereomer wurde in einer Ausbeute von 2,2 g erhalten. Das polarere Diastereomer wurde in einer Ausbeute von 1,6 g erhalten.
$[\alpha]_D^{20}$ = -31,2° (c = 1 in Methanol)

b) N-(1-S-Ethoxycarbonylheptyl)-L-alanin

3,2 g Benzylester (polareres Diastereomer) wurden in 130 ml Ethanol auf 900 mg Palladium-Kohle hydriert. Nach Beendigung der Reaktion wurde vom Katalysator abfiltriert und das Lösungsmittel im Vakuum abdestilliert. Die Ausbeute betrug 2,2 g.
$[\alpha]_D^{20}$ = +9,2° (c = 1 in Methanol)

c) 2-(N-(1-S-Ethoxycarbonylheptyl)-S-alanyl)-(1S,3S,5S)-2-azabicyclo[3.3.0]octan-3-carbonsäurebenzylester

2,3 g der in b) erhaltenen Carbonsäure und 2,3 g (1S,3S,5S)-2-azabicyclo[3.3.0]octan-3-carbonsäurebenzylester wurden in 110 ml absolutem Dimethylformamid gelöst und bei -8 °C mit 6,0 ml Triethylamin und 8,2 ml n-Propylphosphonsäureanhydrid versetzt. Nach einer Rührzeit von 48 Stunden bei Raumtemperatur wurde die Lösung mit Essigester verdünnt und mit gesättigter $NaHCO_3$-Lösung, 10 % Citronensäure und gesättigter NaCl-Lösung gewaschen, getrocknet über $MgSO_4$ und eingeengt. Das Rohprodukt von 3,9 g wurde säulenchromatographisch auf Kieselgel (Cyclohexan/Essigester) gereinigt und ergab 1,2 g reine Substanz.
$[\alpha]_D^{20}$ = -46,6° (c = 1 in Methanol)

d) 2-(N-(1-S-Ethoxycarbonylheptyl)-S-alanyl)-(1S,3S,5S)-2-azabicyclo[3.3.0]octan-3-carbonsäure

1,2 g des obigen Esters (siehe c)) wurden in 50 ml Ethanol über 250 mg Palladium-Kohle hydriert. Nach Beendigung der Reaktion wurde vom Katalysator abfiltriert und die Lösung eingeengt. Die Ausbeute betrug 910 mg.
$[\alpha]_D^{20}$ = +5,3° (c = 1 in Methanol)

e) 400 mg der unter d) erhaltenen Carbonsäure wurden zusamen mit 156 mg 1-Hydroxybenzotriazol und 208 mg Dicyclohexylcarbodiimid in 10 DMF gelöst und über Nacht gerührt. Die Lösung wurde vom angefallenen Dicyclohexylharnstoff abfiltriert, mit Essigester versetzt und mit gesättigter $NaHCO_3$-Lösung, 10 % Citronensäure und gesättigter NaCl-Lösung gewaschen, über $MgSO_4$ getrocknet und eingeengt. Das Rohprodukt wurde mit Petrolether verrührt und die angefallenen Kristalle abgesaugt. Die Ausbeute betrug 170 mg.
$[\alpha]_D^{20}$ = -41,1° (c = 1 in Methanol)

**Beispiel 7**

(3S,5 S,8aS,9aS)-3-Methyl-2N-(1-R-ethoxycarbonylheptyl)-2H-decahydrocyclopenta[4,5]pyrrolo[1,2-a]-pyrazin-1,4-dion

Das in Beispiel 6a) erhaltene unpolarere Diastereomer wurde analog den Beschreibungen 6b)-d) umgesetzt.
550 mg 2-(N-(1-R-Ethoxycarbonylheptyl)-S-alanyl)-(1S,3S,5S)-2-acabicyclo[3.3.0]octan-3-carbonsäure ($[\alpha]_D^{20}$ = +4,4°, (c = 1 in Methanol) wurde zusammen mit 215 mg 1-Hydroxybenzotriazol und 290 mg Dicyclohexylcarbodiimid in 14 ml absolutem Dimethylformamid zur Reaktion gebracht. Nach 48 Stunden Rührzeit bei Raumtemperatur war die Reaktion beendet. Die Aufarbeitung erfolgte wie in Beispiel 6e) beschrieben. Die Ausbeute betrug 330 mg als farbloses Öl.
$[\alpha]_D^{20}$ = -3,7° (c = 1 in Methanol)

**Beispiel 8**

(3S,6S,7aS)-3-Methyl-6-(exo-spiro(bicyclo[2.2.2]octan-2-yl))-2N-(1-S-Ethoxycarbonyl-3-phenylpropyl)-octahydropyrrolo[1,2-a]-pyrazin-1,4-dion

0,4 g N-(1-S-Ethoxycarbonyl-3-phenylpropyl)-S-alanyl-exo-spiro(bicyclo[2.2.2]octan-2,3-pyrrolidin)-5-yl-carbonsäure wurde in 5 ml absolutem DMF zusammen mit 130 mg 1-Hydroxybenzotriazol und 175 mg Dicyclohexylcarbodiimid über Nacht bei Raumtemperatur gerührt. Nach Filtration vom ausgefallenen Harnstoff wurde mit Essigester verdünnt und mit 5 % $NaHCO_3$-Lösung, 10 % Citronensäure, gesättigter NaCl-Lösung gewaschen, anschließend über $MgSO_4$ getrocknet. Das nach Abdestillieren verbliebene Rohprodukt wurde über Kieselgel (Cyclohexan/Essigester) chromatographiert. Die Ausbeute betrug 0,2 g des gewünschten Produktes. Massenspektrum: m/e = 452 $M^+$

**Ansprüche**

1. Verbindung der Formel I

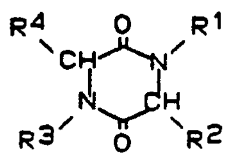

(I),

in welcher
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest

mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^3$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen bedeutet,
$R^4$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatischen-($C_1$-$C_8$)-aliphatischen Rest mit 5 - 12 Ringatomen bedeutet, oder
für

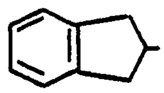

steht oder
$R^3$ und $R^4$ zusammen mit den sie tragenden Atomen ein mono,-bi- oder tricyclisches heterocyclisches Ringsystem mit 3 bis 15 Ring-C-Atomen bilden,
sowie deren physiologische Salze.

2. Verbindung der Formel I gemäß Anspruch 1, in welcher
$R^1$ Wasserstoff;
($C_1$-$C_{18}$)-Alkyl;
einen aliphatisch acyclischen Rest der Formel $C_aH_{(2a+b-1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di- oder tricyclischen, nicht aromatischen, gegebenenfalls verzweigten Kohlenstoffrest der Formel $C_cH_{(2c-d-1)}$ bedeutet, worin c für eine ganze Zahl 3 bis 20 und d für eine ganze Zahl 0 bis (c-2) stehen;
($C_6$-$C_{12}$)-Aryl, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl mono-, di- oder trisubstituiert sein kann;
Amino-($C_1$-$C_8$)-alkyl;
($C_1$-$C_4$)-Alkanoylamino-($C_1$-$C_8$)-alkyl;
($C_7$-$C_{13}$)-Aroylamino-($C_1$-$C_8$)-alkyl;
($C_1$-$C_4$)-Alkoxycarbonylamino-($C_1$-$C_8$)-alkyl;
($C_6$-$C_{12}$)-Aryl-($C_1$-$C_4$)-alkoxycarbonylamino-($C_1$-$C_8$)-alkyl;
($C_6$-$C_{12}$)-Aryl-($C_1$-$C_4$)-alkylamino-($C_1$-$C_8$)-alkyl;
($C_1$-$C_4$)-Alkylamino-($C_1$-$C_8$)-alkyl;
Di-($C_1$-$C_4$)-alkylamino-($C_1$-$C_8$)-alkyl;
Guanidino-($C_1$-$C_8$)-alkyl;
($C_1$-$C_4$)-Alkylthio-($C_1$-$C_8$)-alkyl;
($C_6$-$C_{12}$)-Arylthio-($C_1$-$C_8$)-alkyl oder ($C_6$-$C_{12}$)-Aryloxy-($C_1$-$C_8$)-alkyl, die beide im Arylteil wie oben beschrieben substituiert sein können;
Carboxy-($C_1$-$C_{17}$)-alkyl, ($C_1$-$C_4$)-Alkoxycarbonyl-($C_1$-$C_{17}$)-alkyl, Carbamoyl-($C_1$-$C_{17}$)-alkyl, ($C_1$-$C_4$)-Mono- oder -dialkylcarbamoyl-($C_1$-$C_{17}$)-alkyl, wobei Alkyl gegebenenfalls durch ($C_6$-$C_{12}$)-Aryl substituiert ist, bedeutet;
$R^2$ Wasserstoff,
($C_1$-$C_{18}$)-Alkyl;
einen aliphatischen acyclischen Rest der Formel $C_aH_{(2a-b+1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di-, tri-, tetra- oder pentacyclischen, nicht aromatischen, gegebenenfalls verzweigten Kohlen-

wasserstoffrest der Formel $C_cH_{(2c-d-1)}$, worin c für eine ganze Zahl 3 bis 20 und d für eine gerade Zahl 0 bis (c-2) stehen;
($C_6$-$C_{12}$)-Aryl;
($C_6$-$C_{12}$)-Aryl-($C_1$-$C_8$)-alkyl oder ($C_7$-$C_{13}$)-Aroyl-($C_1$-$C_8$)-alkyl, die im Arylteil wie oben beschrieben substituiert sein können;
mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroarylteil wie oben bei Aryl beschrieben substituiert sein können; oder, falls noch nicht umfaßt, die gegebenenfalls geschützte Seitenkette einer natürlich vorkommenden α-Aminosäure der Formel $R^1$-CH($NH_2$)-COOH bedeutet; und
$R^3$ und $R^4$ zusammen mit den sie tragenden Atomen ein mono-, bi- oder tricyclisches heterocyclisches Ringsystem mit 3 bis 15 Ring-C-Atomen bilden,
sowie deren physiologisch verträgliche Salze.

3. Verbindung der Formel I gemäß eine oder mehreren der Ansprüche 1 bis 2, in welcher
$R^1$ Wasserstoff;
($C_1$-$C_8$)-Alkyl;
($C_2$-$C_6$)-Alkenyl;
($C_3$-$C_9$)-Cycloalkyl;
($C_6$-$C_{12}$)-Aryl, das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann;
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen, das wie in Anspruch 2 bei Aryl beschrieben substituiert sein kann;
Amino-($C_1$-$C_4$)-alkyl;
($C_1$-$C_4$)-Alkanoylamino-($C_1$-$C_4$)-alkyl;
($C_7$-$C_{13}$)-Aroylamino-($C_1$-$C_4$)-alkyl;
($C_1$-$C_4$)-Alkoxycarbonylamino-($C_1$-$C_4$)-alkyl;
($C_6$-$C_{12}$)-Aryl-($C_1$-$C_4$)-alkoxycarbonylamino-($C_1$-$C_4$)-alkyl;
($C_6$-$C_{12}$)-Aryl-($C_1$-$C_4$)-alkylamino-($C_1$-$C_4$)-alkyl;
($C_1$-$C_4$)-Alkylamino-($C_1$-$C_4$)-alkyl;
Di-($C_1$-$C_4$)-alkylamino-($C_1$-$C_4$)-alkyl;
Guanidino-($C_1$-$C_4$)-alkyl;
($C_1$-$C_4$)-Alkylthio-($C_1$-$C_4$)-alkyl;
($C_6$-$C_{12}$)-Arylthio-($C_1$-$C_4$-alkyl, das im Arylteil wie in Anspruch 2 bei Aryl beschrieben substituiert sein kann;
Carboxy-($C_1$-$C_4$)-alkyl;
Carbamoyl-($C_1$-$C_4$)-alkyl;
($C_1$-$C_4$)-Alkoxy-carbonyl-($C_1$-$C_4$)-alkyl;
($C_6$-$C_{12}$)-Aryloxy-($C_1$-$C_4$)-alkyl, das im Arylteil wie in Anspruch 2 bei Aryl beschrieben substituiert sein kann,
bedeutet,
$R^2$ Wasserstoff;
($C_1$-$C_6$)-Alkyl;
($C_2$-$C_6$)-Alkenyl;
($C_2$-$C_6$)-Alkinyl;
($C_3$-$C_9$)-Cycloalkyl;
($C_5$-$C_9$)-Cycloalkenyl;
($C_3$-$C_9$)-Cycloalkyl-($C_1$-$C_4$)-alkyl;
($C_5$-$C_9$)-Cycloalkenyl-($C_1$-$C_4$)-alkyl;
gegebenenfalls teilhydriertes ($C_6$-$C_{12}$)-Aryl, das wie in Anspruch 2 bei $R^1$ beschrieben substituiert sein kann; ($C_6$-$C_{12}$)-Aryl-($C_1$-$C_4$)-alkyl oder ($C_7$-$C_{13}$)-Aroyl-($C_1$ oder $C_2$)-alkyl, die beide wie das vorstehende Aryl substituiert sein können;
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder die gegebenenfalls geschützte Seitenkette einer natürlich vorkommenden α-Aminosäure $R^1$-CH($NH_2$)-COOH bedeutet; und
$R^3$ und $R^4$ die oben angegebene Bedeutung haben sowie deren physiologisch verträgliche Salze.

4. Verbindung der Formel I gemäß einem oder mehreren der Ansprüche 1 bis 3, in welcher
$R^1$ Wasserstoff; ($C_1$-$C_8$)-Alkyl; ($C_2$-$C_6$)-Alkenyl; ($C_3$-$C_9$)-Cycloalkyl; Amino-($C_1$-$C_4$)-alkyl; ($C_2$-$C_5$)-Acylamino-

$(C_1-C_4)$-alkyl; $(C_7-C_{13})$-Aroylamino-$(C_1-C_4)$-alkyl; $(C_1-C_4)$-Alkoxycarbonylamino-$(C_1-C_4)$-alkyl; $(C_1-C_4)$-Alkoxycarbonyl-$(C_1-C_{10})$-alkyl, Carbamoyl-$(C_1-C_{10})$-alkyl, $(C_1-C_4)$-Mono- oder-di-alkylcarbamoyl-$(C_1-C_{10})$-alkyl, wobei Alkyl gegebenenfalls durch Phenyl substituiert ist;
$(C_6-C_{12})$-Aryl-$(C_1-C_6)$-alkyl; $(C_6-C_{12})$-Aryl-$(C_1-C_4)$-alkoxycarbonylamino-$(C_1-C_4)$-alkyl; $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann; bedeutet,
$R^2$ Wasserstoff; $(C_1-C_6)$-Alkyl, das gegebenenfalls durch Amino, $(C_1-C_6)$-Acylamino oder Benzoylamino substituiert sein kann; $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann; $(C_6-C_{12})$-Aryl-$(C_1 - C_4)$-alkyl oder $(C_7-C_{13})$-Aroyl-$(C_1-C_2)$-alkyl, die beide wie vorstehend definiert im Arylrest substituiert sein können; ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen; oder eine Seitenkette einer natürlich vorkommenden, gegebenenfalls geschützten α-Aminosäure, und
$R^3$ und $R^4$ zusammen mit den sie tragenden Atomen ein bi-oder tricyclisches heterocyclisches Ringsystem aus der Reihe Octohydroindol, Octohydrocyclopenta[b]pyrrol, Spiro[(bicyclo[2.2.2]octan)-2,3′-pyrrolidin], 1,2,3,3a,4,6a-Hexahydrocyclopenta[b]pyrrol,Indolin bilden,
sowie deren physiologisch verträgliche Salze.

5. Verfahren zur Herstellung einer Verbindung der Formel I gemäß einem oder mehreren der Ansprüche 1 bis 4, dadurch gekennzeichnet, daß man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, die so erhaltenen Verbindungen intramolekular cyclisiert, Verbindungen der Formel I mit freier(en) Carboxylgruppe(n) gegebenenfalls verestert und die erhaltenen Verbindungen gegebenenfalls in ihre physiologisch verträglichen Salze überführt.

6. Verbindung der Formel I gemäß einem oder mehreren der Ansprüche 1 bis 4 zur Anwendung als Heilmittel.

7. Verbindung der Formel I gemäß einem oder mehreren der Ansprüche 1 bis 4 zur Anwendung als nootropes Mittel.

8. Pharmazeutisches Mittel enthaltend eine wirksame Menge einer Verbindung der Formel I gemäß einem oder mehreren der Ansprüche 1 bis 4 und einen physiologisch unbedenklichen Träger.

9. Verfahren zur Herstellung eines pharmazeutischen Mittels gemäß Anspruch 8, dadurch gekennzeichnet, daß man den Wirkstoff zusammen mit einem Träger und gegebenenfalls weiteren Zusatz- und/oder Hilfsstoffen in eine geeignete Darreichungsform bringt.

Patentansprüche für folgende Vertragsstaaten: GR, ES

1. Verfahren zur Herstellung einer Verbindung der Formel I

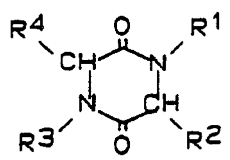

(I),

in welcher
$R^1$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-$(C_1-C_8)$-aliphatischen Rest mit 5 - 12 Ringatomen bedeutet,
$R^2$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,

einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder,
falls nicht von vorstehenden Definitionen schon umfaßt, die erforderlichenfalls geschützte Seitenkette einer natürlich vorkommenden α-Aminosäure bedeutet,
$R^3$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen bedeutet,
$R^4$ Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatischen-($C_1$-$C_8$)-aliphatischen Rest mit 5 - 12 Ringatomen bedeutet, oder
für

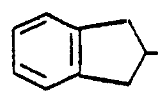

steht oder
$R^3$ und $R^4$ zusammen mit den sie tragenden Atomen ein mono,-bi- oder tricyclisches heterocyclisches Ringsystem mit 3 bis 15 Ring-C-Atomen bilden,
sowie deren physiologische Salze,
dadurch gekennzeichnet, daß
man ihre Fragmente in einem geeigneten Lösungsmittel gegebenenfalls in Gegenwart einer Base und/oder eines Kupplungshilfsmittels miteinander umsetzt, gegebenenfalls intermediär entstehende ungesättigte Verbindungen, wie Schiffsche Basen, reduziert, zum Schutz reaktiver Gruppen temporär eingeführte Schutzgruppen abspaltet, die so erhaltenen Verbindungen intramolekular cyclisiert, Verbindungen der Formel I mit freier(en) Carboxylgruppen(n) gegebenenfalls verestert und die erhaltenen Verbindungen gegebenenfalls in ihre physiologisch verträglichen Salze überführt.

2. Verfahren gemäß Anspruch 1, dadurch gekennzeichnet, daß eine Verbindung hergestellt wird, in der
$R^1$ Wasserstoff;
($C_1$-$C_{18}$)-Alkyl;
einen aliphatisch acyclischen Rest der Formel $C_aH_{(2a+b-1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di- oder tricyclischen, nicht aromatischen, gegebenenfalls verzweigten Kohlenstoffrest der Formel $C_cH_{(2c-d-1)}$ bedeutet, worin c für eine ganze Zahl 3 bis 20 und d für eine ganze Zahl 0 bis (c-2) stehen;
($C_6$-$C_{12}$)-Aryl, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl mono-, di- oder trisubstituiert sein kann;
Amino-($C_1$-$C_8$)-alkyl;
($C_1$-$C_4$)-Alkanoylamino-($C_1$-$C_8$)-alkyl;
($C_7$-$C_{13}$)-Aroylamino-($C_1$-$C_8$)-alkyl;
($C_1$-$C_4$)-Alkoxycarbonylamino-($C_1$-$C_8$)-alkyl;
($C_6$-$C_{12}$)-Aryl-($C_1$-$C_4$)-alkoxycarbonylamino-($C_1$-$C_8$)-alkyl;
($C_6$-$C_{12}$)-Aryl-($C_1$-$C_4$)-alkylamino-($C_1$-$C_8$)-alkyl;
($C_1$-$C_4$)-Alkylamino-($C_1$-$C_8$)-alkyl;
Di-($C_1$-$C_4$)-alkylamino-($C_1$-$C_8$)-alkyl;
Guanidino-($C_1$-$C_8$)-alkyl;

$(C_1-C_4)$-Alkylthio-$(C_1-C_8)$-alkyl;
$(C_6-C_{12})$-Arylthio-$(C_1-C_8)$-alkyl oder $(C_6-C_{12})$-Aryloxy-$(C_1-C_8)$-alkyl, die beide im Arylteil wie oben beschrieben substituiert sein können;
Carboxy-$(C_1-C_{17})$-alkyl, $(C_1-C_4)$-Alkoxycarbonyl-$(C_1-C_{17})$-alkyl, Carbamoyl-$(C_1-C_{17})$-alkyl, $(C_1-C_4)$-Mono- oder di-alkylcarbamoyl-$(C_1-C_{17})$-alkyl, wobei Alkyl gegebenenfalls durch $(C_6-C_{12})$-Aryl substituiert ist, bedeutet;
$R^2$ Wasserstoff,
$(C_1-C_{18})$-Alkyl;
einen aliphatischen acyclischen Rest der Formel $C_aH_{(2a-b+1)}$, worin Doppelbindungen, falls ihre Zahl 1 übersteigt, nicht kumuliert sind, a für eine ganze Zahl 2 bis 18 und b für eine ganze Zahl 2 bis a stehen;
einen mono-, di-, tri-, tetra- oder pentacyclischen, nicht aromatischen, gegebenenfalls verzweigten Kohlenwasserstoffrest der Formel $C_cH_{(2c-d-1)}$, worin c für eine ganze Zahl 3 bis 20 und d für eine gerade Zahl 0 bis (c-2) stehen;
$(C_6-C_{12})$-Aryl;
$(C_6-C_{12})$-Aryl-$(C_1-C_8)$-alkyl oder $(C_7-C_{13})$-Aroyl-$(C_1-C_8)$-alkyl, die im Arylteil wie oben beschrieben substituiert sein können;
mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroarylteil wie oben bei Aryl beschrieben substituiert sein können; oder, falls noch nicht umfaßt, die gegebenenfalls geschützte Seitenkette einer natürlich vorkommenden α-Aminosäure der Formel $R^1$-CH($NH_2$)-COOH bedeutet; und
$R^3$ und $R^4$ zusammen mit den sie tragenden Atomen ein mono-, bi- oder tricyclisches heterocyclisches Ringsystem mit 3 bis 15 Ring-C-Atomen bilden.

3. Verfahren gemäß Anprüche 1 und/oder 2, dadurch gekennzeichnet, daß eine Verbindung hergestellt wird, in der
$R^1$ Wasserstoff;
$(C_1-C_8)$-Alkyl;
$(C_2-C_6)$-Alkenyl;
$(C_3-C_9)$-Cycloalkyl;
$(C_6-C_{12})$-Aryl, das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann; mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen, das wie in Anspruch 2 bei Aryl beschrieben substituiert sein kann;
Amino-$(C_1-C_4)$-alkyl;
$(C_1-C_4)$-Alkanoylamino-$(C_1-C_4)$-alkyl;
$(C_7-C_{13})$-Aroylamino-$(C_1-C_4)$-alkyl;
$(C_1-C_4)$-Alkoxycarbonylamino-$(C_1-C_4)$-alkyl;
$(C_6-C_{12})$-Aryl-$(C_1-C_4)$-alkoxycarbonylamino-$(C_1-C_4)$-alkyl;
$(C_6-C_{12})$-Aryl-$(C_1-C_4)$-alkylamino-$(C_1-C_4)$-alkyl;
$(C_1-C_4)$-Alkylamino-$(C_1-C_4)$-alkyl;
Di-$(C_1-C_4)$-alkylamino-$(C_1-C_4)$-alkyl;
Guanidino-$(C_1-C_4)$-alkyl;
$(C_1-C_4)$-Alkylthio-$(C_1-C_4)$-alkyl;
$(C_6-C_{12})$-Arylthio-$(C_1-C_4$-alkyl, das im Arylteil wie in Anspruch 2 bei Aryl beschrieben substituiert sein kann;
Carboxy-$(C_1-C_4)$-alkyl;
Carbamoyl-$(C_1-C_4)$-alkyl;
$(C_1-C_4)$-Alkoxy-carbonyl-$(C_1-C_4)$-alkyl;
$(C_6-C_{12})$-Aryloxy-$(C_1-C_4)$-alkyl, das im Arylteil wie in Anspruch 2 bei Aryl beschrieben substituiert sein kann, bedeutet,
$R^2$ Wasserstoff;
$(C_1-C_6)$-Alkyl;
$(C_2-C_6)$-Alkenyl;
$(C_2-C_6)$-Alkinyl;
$(C_3-C_9)$-Cycloalkyl;
$(C_5-C_9)$-Cycloalkenyl;
$(C_3-C_9)$-Cycloalkyl-$(C_1-C_4)$-alkyl;
$(C_5-C_9)$-Cycloalkenyl-$(C_1-C_4)$-alkyl;

gegebenenfalls teilhydriertes ($C_6$-$C_{12}$)-Aryl, das wie in Anspruch 2 bei $R^1$ beschrieben substituiert sein kann; ($C_6$-$C_{12}$)-Aryl-($C_1$-$C_4$)-alkyl oder ($C_7$-$C_{13}$)-Aroyl-($C_1$ oder $C_2$)-alkyl, die beide wie das vorstehende Aryl substituiert sein können;

mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder die gegebenenfalls geschützte Seitenkette einer natürlich vorkommenden $\alpha$-Aminosäure $R^1$-$CH(NH_2)$-COOH bedeutet; und

$R^3$ und $R^4$ die in Anspruch 2 angegebene Bedeutung haben.

4. Verfahren gemäß einem oder mehreren der Ansprüche 1 bis 3, dadurch gekennzeichnet, daß eine Verbindung hergestellt wird, in der

$R^1$ Wasserstoff; ($C_1$-$C_8$)-Alkyl; ($C_2$-$C_6$)-Alkenyl; ($C_3$-$C_9$)-Cycloalkyl; Amino-($C_1$-$C_4$)-alkyl; ($C_2$-$C_5$)-Acylamino-($C_1$-$C_4$)-alkyl; ($C_7$-$C_{13}$)-Aroylamino-($C_1$-$C_4$)-alkyl; ($C_1$-$C_4$)-Alkoxycarbonylamino-($C_1$-$C_4$)-alkyl; ($C_1$-$C_4$)-Alkoxycarbonyl-($C_1$-$C_{10}$)-alkyl, Carbamoyl-($C_1$-$C_{10}$)-alkyl, ($C_1$-$C_4$)-Mono- oder-di-alkylcarbamoyl-($C_1$-$C_{10}$)-alkyl, wobei Alkyl gegebenenfalls durch Phenyl substituiert ist;

($C_6$-$C_{12}$)-Aryl-($C_1$-$C_6$)-alkyl; ($C_6$-$C_{12}$)-Aryl-($C_1$-$C_4$)-alkoxycarbonylamino-($C_1$-$C_4$)-alkyl; ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann; bedeutet,

$R^2$ Wasserstoff; ($C_1$-$C_6$)-Alkyl, das gegebenenfalls durch Amino, ($C_1$-$C_6$)-Acylamino oder Benzoylamino substituiert sein kann; ($C_2$-$C_6$)-Alkenyl, ($C_3$-$C_9$)-Cycloalkyl, ($C_5$-$C_9$)-Cycloalkenyl, ($C_3$-$C_7$)-Cycloalkyl-($C_1$-$C_4$)-alkyl, ($C_6$-$C_{12}$)-Aryl oder teilhydriertes Aryl, das jeweils durch ($C_1$-$C_4$)-Alkyl, ($C_1$ oder $C_2$)-Alkoxy oder Halogen substituiert sein kann; ($C_6$-$C_{12}$)-Aryl-($C_1$ - $C_4$)-alkyl oder ($C_7$-$C_{13}$)-Aroyl-($C_1$-$C_2$)-alkyl, die beide wie vorstehend definiert im Arylrest substituiert sein können; ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen; oder eine Seitenkette einer natürlich vorkommenden, gegebenenfalls geschützten $\alpha$-Aminosäure, und

$R^3$ und $R^4$ zusammen mit den sie tragenden Atomen ein bi-oder tricyclisches heterocyclisches Ringsystem aus der Reihe Octohydroindol, Octohydrocyclopenta[b]pyrrol, Spiro[(bicyclo[2.2.2]octan)-2,3′-pyrrolidin], 1,2,3,3a,4,6a-Hexahydrocyclopenta[b]pyrrol,Indolin bilden.

5. Verwendung einer nach einem der Verfahren der Ansprüche 1 bis 4 hergestellten Verbindung der Formel I als Heilmittel.

6. Verwendung einer nach einem der Verfahren der Ansprüche 1 bis 4 hergestellten Verbindung der Formel I als nootropes Mittel.

7. Verfahren zur Herstellung einer pharmazeutischen Zubereitung enthaltend eine nach einem der Verfahren der Ansprüche 1 bis 4 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.

Europäisches Patentamt

# EUROPÄISCHER RECHERCHENBERICHT

Nummer der Anmeldung

EP 89 11 6093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-1 409 185 (GRUPPO LEPETIT S.P.A.)<br>* Ansprüche 1,3,9; Seite 4, Zeilen 3-28 *<br>--- | 1-3,5,6,8 | C 07 D 241/08<br>C 07 D 487/04<br>C 07 D 487/10<br>A 61 K 31/495//<br>(C 07 D 487/04<br>C 07 D 241:00<br>C 07 D 209:00 )<br>(C 07 D 487/10<br>C 07 D 241:00<br>C 07 D 209:00 ) |
| X | DIE PHARMAZIE<br>Band 32, Nr. 1, 1977, Seiten 1-10; S. JOHNE et al.: "Naturstoffe mit Diketopiperazinstruktur"<br>--- | 1-3,5,6 | |
| X | CHEMICAL ABSTRACTS<br>Band 101, Nr. 23, 3. Dezember 1984, Seite 647, Zusammenfassung Nr. 211099f, Columbus, Ohio, USA; E.OCCELLI et al.: "Central nervous system-active substances. Derivatives of octahydro-1,4-dioxopyrrolo (1,2-a)pyrazino-6-carboxylic acids" & Farmaco, Ed. Sci. 1984, Band 39, Nr. 8, Seiten 718-738<br>--- | 1-3,5,6 | |
| X | CHEMICAL ABSTRACTS<br>Band 102, Nr. 13, 1. April 1985, Seite 708, Zusammenfassung nr. 113424j, Columbus, Ohio, USA; N. MARGOUM et al.: "Synthesis of some new thiazolidinopiperazine-2,-5-diones and their pharmacological evaluation" & Eur. J. Med. Chem. - Chim. Ther. 1984, Band 19, Nr. 5, Seiten 415-419<br>--- -/- | 1-3,6 | **RECHERCHIERTE SACHGEBIETE (Int. Cl.5)**<br>C 07 D 241/00<br>C 07 D 487/00 |

Der vorliegende Recherchenbericht wurde für alle Patentansprüche erstellt

| Recherchenort | Abschlußdatum der Recherche | Prüfer |
|---|---|---|
| BERLIN | 28-11-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 89 11 6093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS Band 91 Nr. 3, 16. Juli 1979, Seite 651, Zusammenfassung Nr. 20450d, Columbus, Ohio, USA; Y. KHANDELWAL et al.: "Agents acting on the CNS: Part XXXV. Synthesis of imidazol(1,5-a)pyrazines and pyrazino(1,2-a)pyrazines" & Indian J. Chem. Sect. B, 1978, Band 16B, Nr. 11, Seiten 1015-1018 --- | 1-3,6 | |
| X | JOURNAL OF HETEROCYCLIC CHEMISTRY Band 14, Nr. 4, 1977, Seiten 595-598; M.P. SAXENA et al.: "Preparation and Reactions of some 1,2,3,4-Tetrahydro-2,3-disubstituted 7(or 8)hydroxy-1,4,-dioxopyrazino(1,2-a)indoles" * Seite 595, Schema; Seite 596, Tabelle I; Seite 597, rechte Spalte, untere Hälfte * --- | 1-3,5 | |
| | | | RECHERCHIERTE SACHGEBIETE (Int. Cl.5) |
| X | JOURNAL OF ORGANIC CHEMISTRY Band 35, Nr. 7, 1970, Seiten 2228-2230; N.T. MODI et al.: "Synthesis of 1,2,3,4-Tetrahydro-2,3-disubstituted 10 Hydroxy-1,4-dioxopyrazino(1,2-a)indoles" * Seite 2228, Schemata I,II * --- | 1-3,5 | |
| D,X | EP-A-0 181 152 (FUJISAWA PHARMACEUTICAL CO. LTD.) * Ansprüche * --- | 1,5,6,8 | |
| D,X | EP-A-0 008 186 (BEECHAM GROUP LTD.) * Ansprüche 1,13,15; Beispiele 1-3 * --- -/- | 1,5,6,8 | |

Der vorliegende Recherchenbericht wurde für alle Patentansprüche erstellt

| Recherchenort | Abschlußdatum der Recherche | Prüfer |
|---|---|---|
| BERLIN | 28-11-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 11 6093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 043 219 (ORTHO PHARMACEUTICAL CORP.)<br>* Ansprüche 1,5,6 *<br>--- | 1,5,6,8 | |
| X | EP-A-0 243 122 (FUJISAWA PHARMACEUTICAL CO. LTD.)<br>* Ansprüche 1,4,5; Zusammenfassung *<br>--- | 1,5,6 | |
| D,X | JOURNAL OF PHARMACOLOGY AND EXPERIMENTAL THERAPEUTICS<br>Band 218, Nr. 2, 1981, Seiten 404-408; H.N. BHARGAVA: "Inhibition of Tolerance to the Pharmacological Effects of Human Beta-Endorphin by Prolyl-Leucyl-Glycinamide and Cyclo(Leucylglycine) in the Rat" * Zusammenfassung *<br>--- | 1,6 | |
| D,X | WO-A-8 000 216 (UNIVERSITY OF ILLINOIS FOUNDATION)<br>* Ansprüche 1,2; Seite 10, Zeile 35; Seite 12, Zeilen 26-29 *<br>--- | 1,6 | RECHERCHIERTE SACHGEBIETE (Int. Cl.5) |
| D,X | PHARMACOLOGY BIOCHEMISTRY AND BEHAVIOR<br>Band 10, 1979, Seiten 787-793; T.C. RAINBOW et al.: "Distribution, Survival and Biological Effects in Mice of A Behaviorally Active, Enzymatically Stable Peptide: Pharmacokinetics of Cyclo(Leu-Gly) and Puromycin-induced Amnesia" * Seite 787; Zusammenfassung *<br>--- -/- | 1,6 | |

Der vorliegende Recherchenbericht wurde für alle Patentansprüche erstellt

| Recherchenort | Abschlußdatum der Recherche | Prüfer |
|---|---|---|
| BERLIN | 28-11-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 11 6093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | PHARMACOLOGY BIOCHEMISTRY AND BEHAVIOR Band 8, 1978, Seiten 93-95; J. B. FLEXNER et al.: "ADH and Related Peptides: Effect of Pre- or Posttraining Treatment on Puromycin Amnesia" * Seite 93, Zusammenfassung * --- | 1,6 | |
| X | PATENT ABSTRACTS OF JAPAN Band 7, Nr. 175 (C-179)(1320), 3 August 1983; & JP - A - 58 83682 (GRELAN SEIYAKU K.K.) 19.05.1983 --- | 1,6 | |
| X | EXPERIENTIA Band 29, nr. 5, 1973, Seiten 521,522; G. R. PETTIT et al.: "Isolation and Structural Elucidation of 3,6-Dioxo-Hexahydro-Pyrrolo(1,2-a)-Pyrazine from the Echinoderm Luidia clathrata" --- | 1,6 | |
| X | DE-B-1 301 316 (ASAHI KASEI KOGYO K.K.) * Ansprüche * --- | 1,5 | RECHERCHIERTE SACHGEBIETE (Int. Cl.5) |
| X | TETRAHEDRON LETTERS Nr. 44, 1976, Seiten 3943,3944; R. BAUTE et al.: "Un nouveau metabolite fongique du groupe des epidithio-3,6 dioxo-2,5 piperazines: L'epicorazine A, isolee d?une souche d'epicoccum nigrum link (adelomycetes)" * Seite 3944, Verbindung (I) * --- | 1 | |
| A | US-A-3 194 731 (S. R. SAFIR) * Ansprüche * --- -/- | 1,6,8 | |

Der vorliegende Recherchenbericht wurde für alle Patentansprüche erstellt

| Recherchenort | Abschlußdatum der Recherche | Prüfer |
|---|---|---|
| BERLIN | 28-11-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 89 11 6093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-2 114 230 (WSESOJUSNIJ)<br>* Ansprüche 1,3 *<br>--- | 1,6-8 | |
| A | US-A-3 644 384 (J. W. SCHULENBERG)<br>* Zusammenfassung; Spalte 2, Verbindung IV; Spalte 3, Zeile 68 - Spalte 4, Zeile 34 *<br>----- | 1,6 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| BERLIN | 28-11-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)